# EUROPEAN PATENT SPECIFICATION

(11) **EP 2 289 953 B1**
(45) Date of publication and mention of the grant of the patent: **03.12.2014**
(21) Application number: 09766732.3
(22) Date of filing: 15.06.2009
(51) Int. Cl.: C08F 16/32, C07C 43/16, C08F 2/44, C08G 65/18, C08L 33/00, C08L 63/00, C09D 5/00, C09D 11/00, C09D 129/10, C09J 129/10

(54) **MULTIFUNCTIONAL VINYL ETHER AND RESIN COMPOSITION CONTAINING SAME**
MULTIFUNKTIONELLER VINYLETHER UND HARZZUSAMMENSETZUNG DAMIT
ÉTHER VINYLIQUE MULTIFONCTIONNEL ET COMPOSITION DE RÉSINE LE CONTENANT

(30) Priority: 18.06.2008 JP 2008158889
(43) Date of publication of application: 02.03.2011
(73) Proprietor: Nippon Carbide Industries Co., Inc., Minato-ku, Tokyo 108-8466 (JP)
(72) Inventor: TAKAMATSU, Hiroaki, Uozu-shi Toyama 937-0801 (JP); YAMAMOTO, Kyoko, Namerikawa-shi Toyama 936-8555 (JP); YAMAGATA, Noboru, Namerikawa-shi Toyama 936-8555 (JP)
(74) Representative: Becker Kurig Straus
(86) International application number: PCT/JP2009/061253
(87) International publication number: WO 2009/154284

(56) References cited:
- EP-A1- 2 230 283
- WO-A1-99/10303
- JP-A- 7 005 684
- JP-A- 7 043 924
- JP-A- 10 130 185
- JP-A- 10 139 708
- JP-A- 2001 081 055
- JP-A- 2005 113 109
- JP-A- 2005 170 996
- JP-A- 2008 105 417
- US-A- 5 095 154

## Description

### TECHNICAL FIELD

The present invention relates to a polyfunctional vinyl ether, which is low in skin irritability, low in odor and excellent in dissolution capability for a polymerization initiator. The present invention further relates to a polyfunctional vinyl ether resistant to the effects of oxygen at the time of polymerization and excellent in curability.

### BACKGROUND ART

Conventionally, acrylic compounds have been generally used as the base compounds of resin compositions in many fields such as inks, paints, adhesives, resists. However, when using an acrylic compound as a base compound, there are problems of skin irritability and odor and of workability such as the inhibition of curing due to oxygen. In recent years, greater functions have been demanded for resins. It is becoming no longer possible to sufficiently meet the needs by acrylic compounds. On the other hand, vinyl ether compounds are low in skin irritability, low in odor and exhibit quick curability in cationic polymerization, while resin compositions using the same are characterized by resistant to the effects of oxygen at the time of curing. However, vinyl ether compounds generally have the problem of a low dissolving capability for polymerization initiators. Therefore, vinyl ether compounds which can dissolve a polymerization initiator well, are low in skin irritation, are low in odor, are resistant to the effects of oxygen at the time of polymerization and is excellent in curability has been sought.

### PRIOR ART LIST

### Patent Literature

PLT 1: Japanese Patent Publication No. 7-5684A

### SUMMARY OF INVENTION

### Problem to be Solved By Invention

Accordingly, an object of the present invention is to provide a polyfunctional vinyl ether having low skin irritability, low odor, excellent dissolving capability for a polymerization initiator, excellent resistivity against the effects of oxygen at the time of polymerization and excellent curability and a resin composition comprising the same.

### Means for Solving Problem

The present inventors found that polyfunctional vinyl ether represented by the following formula (I) dissolves a polymerization initiator well: 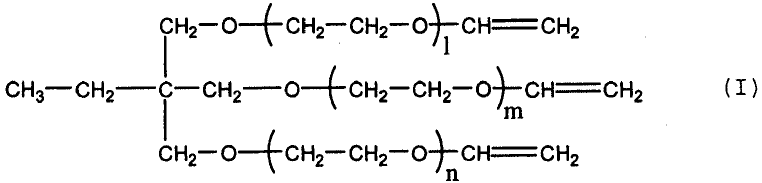 where 1, m and n in the above formula (I) are integers satisfying 1 + m + n > 3.

Further, there is a report relating to vinyl ether of the above formula (I) where (1, m, n)=(1, 1, 1) (see PLT 1), but this literature neither describes nor suggests at all the dissolving capability for a polymerization initiator.

On the other hand, the present inventors found that a polyfunctional vinyl ether satisfying 1 + m + n > 3 in the above formula (I) can dissolve a polymerization initiator well and that a resin composition using the same has a superior curability.

### Effects of Invention

The polyfunctional vinyl ether represented by the following formula (I) in the present invention has low skin irritability, low odor, excellent dissolving capability for a polymerization initiator and excellent resistivity to the effects of oxygen at the time of curing. Further, the resin composition comprising the polyfunctional vinyl ether (I) of the present invention dissolves a polymerization initiator well and is superior in the curability, and, therefore, can be used in inks, paints, adhesives, resists, and many other fields: 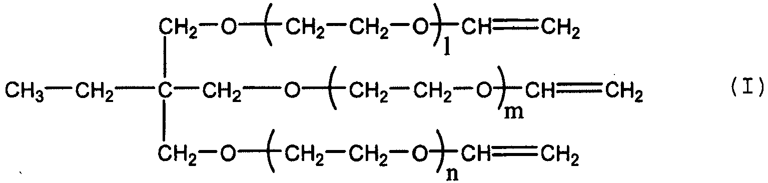 where 1, m and n in the above formula (I) are integers satisfying 1 + m + n > 3.

### BRIEF DESCRIPTION OF DRAWINGS

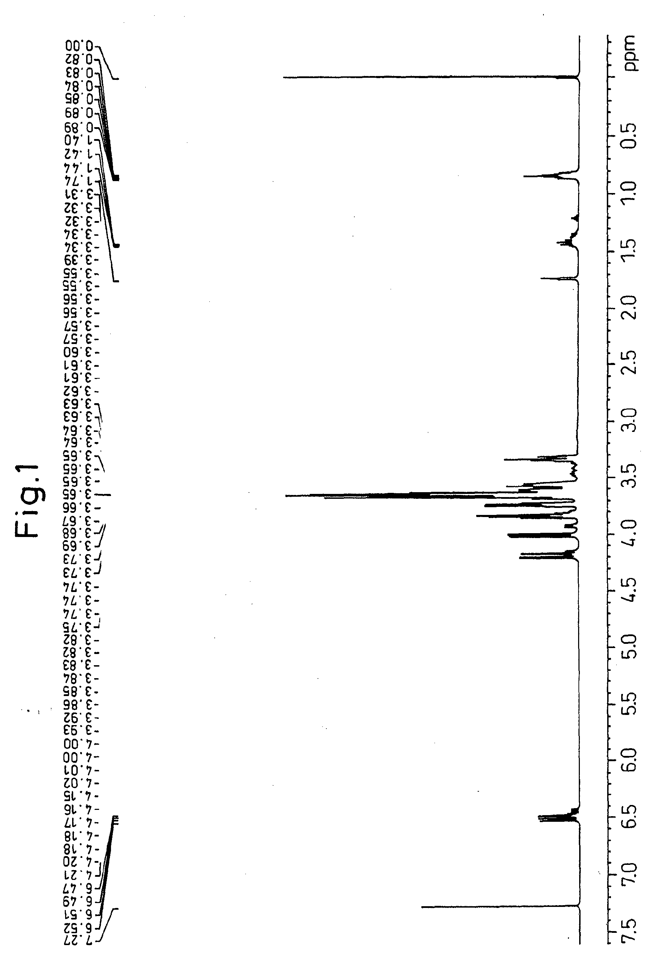

FIG. 1 shows an ¹HNMR spectrum of the trimethylolpropane ethyloxy vinyl ether of Example 6.

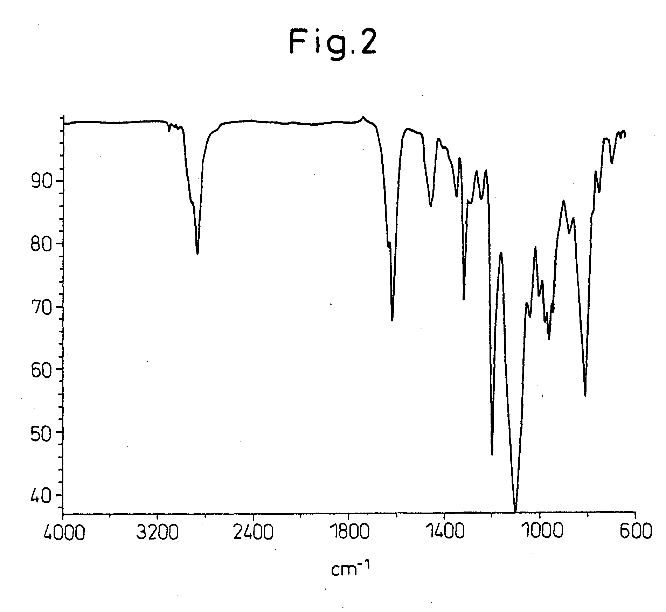

FIG. 2 shows an FT-IR spectrum of the trimethylolpropane ethyloxy vinyl ether of Example 6.

### EMBODIMENTS OF INVENTION

The singular forms ("a", "an", "the") used in the Description and the attached Claims should be understood as including the plural forms, except when otherwise clear from the context.

As the starting material of the polyfunctional vinyl ether of the present invention represented by the above formula (I), it is possible to use a trimethylolpropane ethyloxylated compound such as of the following formula (II) commercially available as industrial materials such as a surfactant, resin modifier, etc. Further, if desired, it is also possible to use these commercially available materials after further purification. 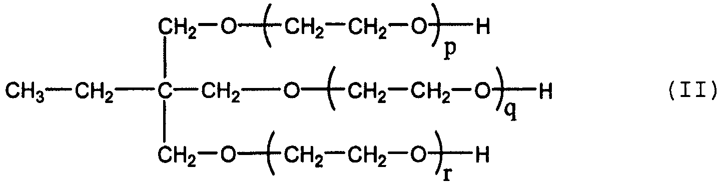 where p, q and r in the above formula (II) are integers satisfying p + q + r > 3.

The polyfunctional vinyl ether represented by the above formula (I) in the present invention preferably satisfies l + m + n ≥ 5, more preferably l + m + n ≥ 7, particularly preferably l + m + n ≥ 9, from the viewpoint of the dissolving capability to a polymerization initiator. Therefore, as a preferable starting material of a polyfunctional vinyl ether of the present invention, there is a trimethylolpropane ethyloxylated compound satisfying p + q + r ≥ 5, more preferably p + q + r ≥ 7, particularly preferably p + q + r ≥ 9 in the above formula (II).

The production method of the polyfunctional vinyl ether according to the present invention is not particularly limited, but when using a trimethylolpropane ethyloxylated compound represented by the above formula (II), a known method may be used. That is, the method may be used of reacting an trimethylolpropane ethyloxyl adduct in the presence of an alkali metal compound such as potassium hydroxide, sodium hydroxide in a solution of a solvent such as dimethyl sulfoxide (DMSO), dimethylimidazolidinone (DMI) in an acetylene atmosphere. Further, a vinyl group-exchange reaction (see Japanese Patent Publication No. 5-221908A, Japanese Patent Publication No. 2003-73321A, J. Org. Chem. 2003, 68, 5225-, etc.), and the like may also be used.

Specifically describing, a preferred production method of a polyfunctional vinyl ether according to the present invention, a pressure resistant reaction vessel made of stainless steel (SUS) is charged with, as a solvent, dimethyl sulfoxide, then is charged with a trimethylolpropane ethyloxyl adduct and further charged with a reaction catalyst (e.g., potassium hydroxide). The addition amount of the reaction catalyst is not particularly limited, but is preferably, based upon 1.0 mol of the starting alcohol, 0.05 to 1.0 mol, more preferably 0.20 to 0.50 mole.

Then, after, the inner volume of the reaction vessel is replaced with nitrogen gas, the reaction vessel is sealed and acetylene is pumped in, while increasing the temperature, to cause a reaction, whereupon the polyfunctional vinyl ether of the present invention, that is, a trimethylolpropane ethyloxy vinyl ether, is formed. The reaction conditions are not particularly limited, but an acetylene pressure of 0.01 to 0.18 MPa and a reaction temperature of 80 to 180°C is preferred. The more preferable acetylene pressure is 0.15 to 0.18 MPa, while the reaction temperature is 90 to 150°C.

The reaction time is not particularly limited, but the consumption amount of acetylene is decreased when the reaction is closer to end point, so it is possible to learn the time of the end of the reaction.

After the end of the reaction, the reaction solution is extracted, washed with water and alkali component and high boiling point solvents are removed to thereby obtain a crude product. The product may be purified by suitably combining known purification and separation methods such as vacuum distillation, column chromatography, etc., thereby to obtain the polyfunctional vinyl ether according to the present invention.

Then, a resin composition using the polyfunctional vinyl ether of the present invention will be explained. The polyfunctional vinyl ether of the present invention has low skin irritability, low odor and superior dissolving capability for a polymerization initiator, while a resin composition using a polyfunctional vinyl ether of the present invention has superior curability, and, therefore, can be used for numerous fields such as inks, paints, adhesives, resists, etc. For example, when used for inks, paints, etc., it is possible to formulate, into a resin component selected from acryls, epoxys, oxetanes, etc. a polyfunctional vinyl ether of the present invention and a polymerization initiator, and, further, if desired, a solvent, dye, pigment, plasticizer, cross-linking agent, filler and various other polymerizable compounds, to obtain the desired resin composition.

The formulating ratio of the resin component and the polyfunctional vinyl ether in the resin composition of the present invention is not particularly limited, but, from the viewpoint of the curing rate or hardness or smoothness of the cured product, it is preferable to use the polyfunctional vinyl ether in an amount of 1 to 200 parts by weight, more preferably 3 to 120 parts by weight, still more preferably 10 to 100 parts by weight, based upon 100 parts by weight of the resin component.

The polymerization initiator may be either a heat polymerization initiator or a light polymerization initiator. It is not particularly limited so long as a radical polymerization or ion polymerization can be initiated. The addition amount of the polymerization initiator, based upon the weight of the polyfunctional vinyl ether of the present invention, is usually 0.01 to 30% by weight, preferably 0.01 to 20% by weight. The polymerization initiator may be directly mixed with the polyfunctional vinyl ether of the present invention or may be mixed with the resin composition.

### EXAMPLES

Examples will now be given to explain the present invention in further detail, but the present invention is by no means limited to these Examples in any event.

In the following Examples and Comparative Examples, the compounds were confirmed by measurement by nuclear magnetic resonance (NMR) spectrums and by infrared (IR) spectrums.

### EXAMPLE 1

A 300 ml SUS pressure resistant reaction vessel equipped with a stirrer, pressure gauge, thermometer, gas introduction pipe and gas purge line was charged with 150 g of dimethyl sulfoxide, 50 g of trimethylolpropane ethyloxylated alcohol of the above formula (II), wherein p + q + r = 4, then, as a catalyst, potassium hydroxide with a purity 95% by weight in 30 mol%, based upon the starting alcohol, was added. The solution was stirred, while introducing nitrogen gas to replace the atmosphere inside the reaction vessel with nitrogen, then the reaction vessel was sealed and the reaction vessel was filled with acetylene gas at about 0.18 MPa pressure. Then, while maintaining the gauge pressure at about 0.18 MPa, the temperature inside the reaction vessel was increased to 120°C. At the reaction temperature of 120°C, the reaction was continued until the consumption of acetylene was decreased. During that time, acetylene gas was successively replenished to maintain the pressure inside the reaction vessel constantly at about 0.18 MPa. After the end of the reaction, the remaining acetylene gas was purged to obtain 209 g of the reaction solution. Thereafter, the reaction solution was extracted with diethyl ether and rinsed with water and the diethyl ether was distilled off to obtain 52 g (yield 83%) of the polyfunctional vinyl ether of the present invention, wherein 1 + m + n = 4).

### EXAMPLE 2

Except for changing the starting material used to 50 g of trimethylolpropane ethyloxylated alcohol having the above formula (II) wherein p + q + r = 5, the same reaction as in Example 1 was carried out. After the end of the reaction, the remaining acetylene gas was purged to obtain 207 g of the reaction solution. Thereafter, the reaction solution was extracted with diethyl ether and rinsed with water and the diethyl ether was distilled off to obtain 50 g (yield 82%) of the polyfunctional vinyl ether of the present invention having the above formula (I) wherein 1 + m + n = 5.

### EXAMPLE 3

Except for changing the starting material used to 50 g of trimethylolpropane ethyloxylated alcohol having the above formula (II) wherein p + q + r = 6, the same reaction as in Example 1 was carried out. After the end of the reaction, the remaining acetylene gas was purged to obtain 207 g of the reaction solution, Thereafter, the reaction solution was extracted with diethyl ether and rinsed with water and the diethyl ether was distilled off to obtain 52 g (yield 87%) of the polyfunctional vinyl ether of the present invention having the above formula (I), wherein 1 + m + n = 6.

### EXAMPLE 4

Except for changing the starting material used to 50 g of trimethylolpropane ethyloxylated alcohol having the above formula (II) wherein p + q + r = 7, the same reaction as in Example 1 was carried out. After the end of the reaction, the remaining acetylene gas was purged to obtain 202 g of the reaction solution. Thereafter, the reaction solution was extracted with diethyl ether and rinsed with water and the diethyl ether was distilled off to obtain 49 g (yield 83%) of the polyfunctional vinyl ether of the present invention having the above formula (I) wherein 1 + m + n = 7.

### EXAMPLE 5

Except for changing the starting material used to 50 g of trimethylolpropane ethyloxylated alcohol having the above formula (II) wherein p + q + r = 8, the same reaction as in Example 1 was carried out. After the end of the reaction, the remaining acetylene gas was purged to obtain 205 g of the reaction solution. Thereafter, the reaction solution was extracted with diethyl ether and rinsed with water and the diethyl ether was distilled off to obtain 46 g (yield 79%) of the polyfunctional vinyl ether of the present invention having the above formula (I) wherein 1 + m + n = 8.

### EXAMPLE 6

Except for changing the starting material used to 50 g of trimethylolpropane ethyloxylated alcohol having the above formula (II) wherein p + q + r = 9, the same reaction as in Example 1 was carried out. After the end of the reaction, the remaining acetylene gas was purged to obtain 204 g of the reaction solution. Thereafter, the reaction solution was extracted with diethyl ether and rinsed with water and the diethyl ether was distilled off to obtain 43 g (yield 76%) of the polyfunctional vinyl ether of the present invention having the above formula (I) wherein 1 + m + n = 9.

### EXAMPLE 7

Except for changing the starting material used to 50 g trimethylolpropane ethyloxylated alcohol having the above formula (II) wherein p + q + r ≥ 6, the same reaction as in Example 1 was carried out. After the end of the reaction, the remaining acetylene gas was purged to obtain 200 g of the reaction solution. Thereafter, the reaction solution was extracted with diethyl ether and rinsed with water and the diethyl ether was distilled off to obtain 48 g of the polyfunctional vinyl ether of the present invention having the above formula (I) wherein 1 + m + n ≥ 6.

### EXAMPLE 8

Except for changing the solvent used to DMI, the same reaction as in Example 3 was carried out. After the end of the reaction, the remaining acetylene gas was purged to obtain 217 g of the reaction solution. Thereafter, the reaction solution was extracted with diethyl ether and rinsed with water and the diethyl ether was distilled off to obtain 44 g (yield 73%) of the polyfunctional vinyl ether of the present invention having the above formula (I) wherein 1 + m + n = 6.

### EXAMPLE 9

A 342 mg (0.51 mmol) amount of [Ir(cod)Cl]₂ (di-µ-chlorobis(1,5-cyclooctadiene)II iridium) and 3.2 g (30.0 mmol) of sodium carbonate; were mixed with 30 ml of toluene, 5.0 g of trimethylolpropane ethyloxylated alcohol having the above formula (II) wherein p + q + r = 6 and 8.6 mg; (100.2 mmol) of vinyl acetate were added, the solution was stirred under a nitrogen atmosphere at 100°C for 3.5 hours, then the reaction solution was extracted with diethyl ether and rinsed with water and the diethyl ether was distilled off to obtain 2.4 g (yield 39%) of the polyfunctional vinyl ether of the present invention having the above formula (I) wherein 1 + m + n = 6.

### COMPARATIVE EXAMPLE 1

Except for changing the starting material used to 50 g of a trimethylolpropane ethyloxylated alcohol having the above formula (II) wherein p + q + r = 3, the same reaction as in Example 1 was carried out. After the end of the reaction, the remaining acetylene gas was purged to obtain 218 g of the reaction solution. Thereafter, the reaction solution was extracted with diethyl ether and rinsed with water and the diethyl ether was distilled off to obtain 56.2 g (yield 87%) of the polyfunctional vinyl ether having the following formula (III):

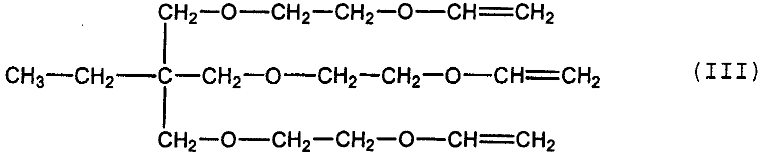

The polyfunctional vinyl ethers produced in Examples 1 to 7 and Comparative Example 1 were used to evaluate the dissolving capability for various types of polymerization initiators and the curability of resin compositions.

### TEST EXAMPLE 1

To 1.0 g of the polyfunctional vinyl ether, obtained in each of Examples 1 to 7, the polymerization initiator described in Table I was added and the solubility was evaluated. Note that, as Comparative Example 2, trimethylolpropane trivinyl ether not having ethylene oxide parts was similarly evaluated. The results are shown in the following Table I:

**Table I**

| | | TS-91 | | | | | CPI-110P | | | | | Irgacure 250 | | | | |
|---|---|---|---|---|---|---|---|---|---|---|---|---|---|---|---|---|
| | wt% | 0.1 | 0.5 | 1.0 | 1.5 | 2.0 | 0.1 | 0.5 | 1.0 | 1.5 | 2.0 | 0.1 | 0.5 | 1.0 | 1.5 | 2.0 |
| Example 1 | | G | G | P | P | P | G | G | P | P | P | G | G | G | G | G |
| Example 2 | | G | G | G | P | P | G | G | P | P | P | G | G | G | G | G |
| Example 3 | | G | G | G | G | P | G | G | G | P | P | G | G | G | G | G |
| Example 4 | | G | G | G | G | G | G | G | G | G | P | G | G | G | G | G |
| Example 5 | | G | G | G | G | G | G | G | G | G | P | G | G | G | G | G |
| Example 6 | | G | G | G | G | G | G | G | G | G | G | G | G | G | G | G |
| Example 7 | | G | G | G | G | G | G | G | G | G | P | G | G | G | G | G |
| Comparative Example 1 | | G | G | P | P | P | G | P | P | P | P | G | G | G | G | G |
| Comparative Example 2 | | P | P | P | P | P | P | P | P | P | P | P | P | P | P | P |

| | | | | | | | | | | | | | | | | |
|---|---|---|---|---|---|---|---|---|---|---|---|---|---|---|---|---|
| G: good (dissolved in several minutes to several days) P: poor (even after several days, there were undissolved components or layer separation) | | | | | | | | | | | | | | | | |

As the polymerization initiators in the above formulations, TS-91; solid state (made by Sanwa Chemical), CPI-110P; solid state (made by San-Apro) and Irgacure 250; solution state (made by Ciba Specialty Chemicals) were used.

### TEST EXAMPLE 2

A resin composition comprising each polyfunctional vinyl ether compound obtained in Examples 1 to 7, to which 0.5% by weight of CPI-110P was added, was subjected to a curing test in the air using a QRM-2023-D-00 high voltage mercury lamp UV irradiation apparatus (made by Orc Manufacturing Co., Ltd.), whereupon a good cured product was able to be obtained by one pass (about 118 mJ/cm²).

### TEST EXAMPLE 3

The viscosity of each polyfunctional vinyl ether compound obtained in Examples 1 to 7 was measured by a rheometer (25°C). The results were a low value of 15 to 65 mPa·s.

In particular, the viscosity of the polyfunctional vinyl ether of the present invention obtained in Example 3 having the formula (I) wherein 1 + m + n = 6 was 39.8 mPa·s and lower than the 65mPa·s of the triacrylate compound having equivalent ethylene oxide parts. This can be utilized for inks requiring a low viscosity resin composition, in particular ink for ink jet use.

### INDUSTRIAL APPLICABILITY

The polyfunctional vinyl ether of the present invention has low skin irritability, low odor and excellent dissolving capability for a polymerization initiator, and therefore, can be used in numerous fields such as inks, paints, adhesives, resists.

## Claims

1. A polyfunctional vinyl ether represented by the following formula (I): 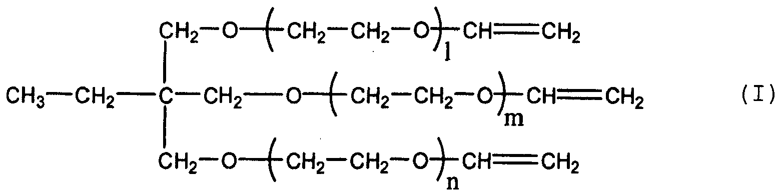 wherein 1, m and n in the above formula (I) indicate integers satisfying 1 + m + n > 3.

2. A polyfunctional vinyl ether as claimed in claim 1, wherein 1, m and n satisfy 1 + m + n ≥ 5.

3. A polyfunctional vinyl ether as claimed in claim 2, wherein 1, m and n satisfy 1 + m + n = 9.

4. A resin composition comprising a polyfunctional vinyl ether as claimed in any one of claims 1 to 3.

5. A resin composition as claimed in claim 4, wherein said resin composition comprises at least one resin component selected from acryls, epoxys and oxetanes.

## Patentansprüche

1. Polyfunktionaler Vinylether, dargestellt durch die folgende Formel (I): 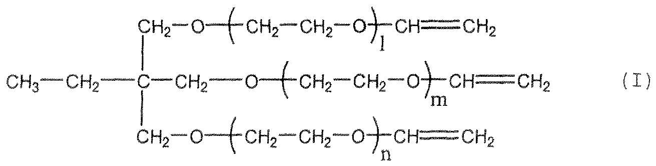 worin 1, m und n in der vorstehenden Formel (I) ganze Zahlen darstellen, die der Bedingung 1 + m + n > 3 genügen.

2. Polyfunktionaler Vinylether nach Anspruch 1, worin 1, m und n der Bedingung 1 + m + n ≥ 5 genügen.

3. Polyfunktionaler Vinylether nach Anspruch 2, worin 1, m und n der Bedingung 1 + m + n = 9 genügen.

4. Harzzusammensetzung, welche einen polyfunktionalen Vinylether nach einem der Ansprüche 1 bis 3 umfasst.

5. Harzzusammensetzung nach Anspruch 4, worin die Harzzusammensetzung mindestens eine Harzkomponente umfasst, ausgewählt unter Acrylen, Epoxiden und Oxetanen.

## Revendications

1. Vinyléther polyfonctionnel représenté par la formule (I) suivante : 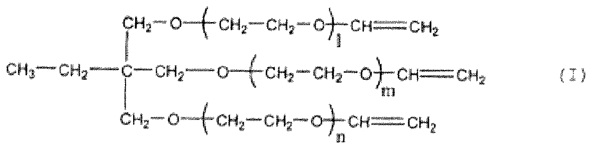 dans laquelle l, m et n dans la formule (I) ci-dessus désignent des entiers satisfaisant à l + m + n > 3.

2. Vinyléther polyfonctionnel selon la revendication 1, l, m et n satisfaisant à l + m + n ≥ 5.

3. Vinyléther polyfonctionnel selon la revendication 2, l, m et n satisfaisant à l + m + n = 9.

4. Composition de résine comprenant un vinyléther polyfonctionnel selon l'une quelconque des revendications 1 à 3.

5. Composition de résine selon la revendication 4, ladite composition de résine comprenant au moins un constituant de résine choisi parmi les acryliques, les époxys et les oxétanes.
